# EUROPEAN PATENT APPLICATION

(11) **EP 4 386 083 A1**
(43) Date of publication of application: **19.06.2024**
(21) Application number: 22855910.0
(22) Date of filing: 10.08.2022
(51) Int. Cl.: C12N 5/077

(54) **METHOD FOR PRODUCING RENAL INTERSTITIAL PROGENITOR CELLS, ERYTHROPOIETIN-PRODUCING CELLS, AND METHOD FOR PRODUCING RENIN-PRODUCING CELLS**

(30) Priority: 11.08.2021 US 202163232102 P; 12.04.2022 US 202263362846 P
(71) Applicant: Kyoto University, Kyoto-shi, Kyoto 606-8501 (JP); Rege Nephro Co., Ltd., Kyoto-shi, Kyoto, 606-8501 (JP)
(72) Inventor: OSAFUNE, Kenji, Kyoto-shi, Kyoto 606-8501 (JP); TSUJIMOTO, Hiraku, Kyoto-shi, Kyoto 606-8501 (JP)
(74) Representative: Hoffmann Eitle
(86) International application number: PCT/JP2022/030664
(87) International publication number: WO 2023/017848

(57) **Abstract**

A method of producing renal interstitial progenitor cells, the method including the step of culturing HOXC10-positive cells in a medium comprising a GSK (glycogen synthase kinase)-3β inhibitor, a SHH (Sonic Hedgehog) signal activator, and a TGF (transforming growth factor) β inhibitor, and preferably also comprising IL (interleukin)-1β and retinoic acid, to induce the renal interstitial progenitor cells.

## Description

### TECHNICAL FIELD

The present invention relates to a method of differentiation induction from HOXC10-positive cells into renal interstitial progenitor cells. The present invention also relates to a method of differentiation induction from renal interstitial progenitor cells into renal erythropoietin-producing cells, or into renin-producing cells such as mesangial cells and juxtaglomerular cells.

### BACKGROUND ART

The number of patients with chronic kidney diseases (CKDs) in Japan is estimated to be about 13 million at present, and these diseases are called a new national disease. Only a limited number of curative treatment methods are available for chronic kidney diseases, and there are not less than 340,000 patients with end-stage chronic renal failure who require dialysis treatment due to progression of the diseases. This has been a serious problem not only from the medical point of view, but also from the viewpoint of medical economy. An example of the curative treatment for chronic kidney diseases, including end-stage chronic renal failure, is kidney transplantation. However, since the supply of donor organs is seriously insufficient, their demand has not been satisfied at all.

The kidney is derived from the intermediate mesoderm, which is a tissue in the early fetal period. In vertebrates, three kidney primordia: the pronephros, the mesonephros, and the metanephros, are formed from the intermediate mesoderm, and, in mammals, the metanephros becomes the adult kidney. The metanephros develops as a result of interaction between a tissue called metanephric mesenchyme, which differentiates into nephrons and interstitium of the adult kidney in the future, and a tissue called ureteric bud, which differentiates into collecting ducts, the renal pelvis and the ureters.

If an efficient method of inducing differentiation from pluripotent stem cells such as induced pluripotent stem (iPS) cells or embryonic stem (ES) cells into cells constituting the kidney is established, the method can be expected to be useful, in the future, for three-dimensional reconstruction of a kidney as a solution to the insufficiency of donors in kidney transplantation, and for providing sources of glomeruli and tubular cells in cell therapy. Furthermore, such a method can be expected to enable development of assay systems for nephrotoxicity of agents, which systems use glomeruli and tubular cells or kidney tissues containing these, and preparation of disease models and research for development of therapeutic agents and the like using kidney cells and kidney tissues prepared from disease-specific iPS cells.

The group of the present inventors has reported a method of differentiation induction from pluripotent stem cells into nephron progenitor cells (Patent Documents 1 and 2).

On the other hand, formation of the complex, tree-like structure of the kidney, especially the tree-like structure of the collecting ducts, has been thought to require signals from interstitial cells. Several groups have reported utilization of the interaction between the anterior and posterior intermediate mesoderm cells differentiated from human pluripotent stem cells, for preparation of a multilineage kidney organoid containing interstitial cells (Non-Patent Documents 1 and 2). However, selective induction of renal interstitial progenitor cells from human pluripotent stem cell has not been realized.

### PRIOR ART DOCUMENTS

### [Patent Documents]

[Patent Document 1] WO 2014/200115
[Patent Document 2] WO 2018/216743

### [Non-patent Documents]

[Non-patent Document 1] Takasato et al., Nature volume 526, pages 564-568 (2015)
[Non-patent Document 2] Uchimura et al., Cell Rep. 2020 Dec 15;33(11):108514.

### SUMMARY OF THE INVENTION

### PROBLEMS TO BE SOLVED BY THE INVENTION

In the recent research of regenerative medicine using pluripotent stem cells, cells and organoids of multiple kidney-lineage cells have been developed. However, formation of more functional segments of the kidney requires a variety of interactions of epithelial cells and interstitial cells, and hence a method of selectively inducing renal interstitial progenitor cells from pluripotent stem cells has been demanded.

Accordingly, an object of the present invention is to provide a method of efficiently inducing differentiation into renal interstitial progenitor cells. Another object is to provide a method of differentiation induction from renal interstitial progenitor cells into renal EPO-producing cells, and into renin-producing cells such as mesangial cells and juxtaglomerular cells.

### MEANS FOR SOLVING THE PROBLEMS

The present inventors intensively studied to solve the above problem. As a result, the present inventors discovered that, by culturing HOXC10-positive cells in a medium containing a GSK-3β inhibitor and a TGFβ inhibitor, and preferably also containing a SHH signal activator, IL-1β, and retinoic acid, selective differentiation induction into renal interstitial progenitor cells (IPCs) is possible. As a result of in vivo transplantation of a mixture of the induced IPCs and nephron progenitor cells (NPCs) in mice, a mesangium-like structure was formed. Further, by identifying differentiation induction factors by screening, IPCs derived from pluripotent stem cells could be induced to differentiate into the lineages of renin-producing cells such as mesangial cells and juxtaglomerular cells, and of renal erythropoietin (EPO)-producing cells in vitro. The present invention was completed based on such a discovery.

More specifically, the present invention has the following characteristics.
[1] A method of producing renal interstitial progenitor cells, the method comprising the step of culturing HOXC10-positive cells in a medium comprising a GSK (glycogen synthase kinase)-3β inhibitor, a SHH (Sonic Hedgehog) signal activator, and a TGF (transforming growth factor) β inhibitor, to induce the renal interstitial progenitor cells.
[2] The method of producing renal interstitial progenitor cells according to [1], wherein the medium further comprises IL (interleukin)-1β.
[3] The method of producing renal interstitial progenitor cells according to [2], further comprising the following steps 1) and 2):
   1) the step of culturing HOXC10-positive cells in a medium comprising a GSK-3β inhibitor, a SHH signal activator, a TGFβ inhibitor, and IL-1β; and
   2) the step of subsequently performing culture in a medium comprising a GSK-3β inhibitor, a SHH signal activator, and a TGFβ inhibitor.
[4] The method of producing renal interstitial progenitor cells according to any one of [1] to [3], wherein the medium further comprises retinoic acid.
[5] The method of producing renal interstitial progenitor cells according to any one of [1] to [4], wherein the culture is carried out by suspension culture.
[6] The method of producing renal interstitial progenitor cells according to any one of [1] to [5], wherein the GSK-3β inhibitor is CHIR99021; the SHH signal activator is SHH protein, purmorphamine, or SAG (Smoothened Agonist); and the TGFβ inhibitor is SB431542, A83-01, or LDN193189.
[7] The method of producing renal interstitial progenitor cells according to any one of [1] to [6], wherein the HOXC10-positive cells are HOXC10-positive cells induced by differentiation from pluripotent stem cells.
[8] The method according to [7], wherein the HOXC10-positive cells are HOXC10-positive cells produced by a method comprising the following steps (i) to (iv):
   (i) the step of culturing the pluripotent stem cells in a medium comprising bFGF (basic fibroblast growth factor), BMP (bone morphogenetic protein) 4, a GSK-3β inhibitor, and retinoic acid or a derivative thereof;
   (ii) the step of culturing the cells obtained in Step (i), in a medium comprising bFGF, a GSK-3β inhibitor, and BMP7;
   (iii) the step of culturing the cells obtained in Step (ii) in a medium comprising a GSK-3β inhibitor and a TGFβ inhibitor; and
   (iv) the step of culturing the cells obtained in Step (iii), in a medium comprising bFGF, a GSK-3β inhibitor, activin, and a ROCK inhibitor.
[9] The method according to [7] or [8], wherein the pluripotent stem cells are induced pluripotent stem (iPS) cells.
[10] A renal interstitial progenitor cells produced by the method according to any one of [1] to [9].
[11] A pharmaceutical composition comprising the renal interstitial progenitor cells according to [10].
[12] The pharmaceutical composition according to [11], further comprising a nephron progenitor cell.
[13] A method of producing renal EPO (erythropoietin)-producing cells, the method comprising the step of culturing renal interstitial progenitor cells in a medium comprising a GSK-3β inhibitor, a SHH signal activator, and a HIF (hypoxia-inducible factor) inhibitor, to produce the renal EPO-producing cells.
[14] The method of producing renal EPO-producing cells according to [13], wherein the medium further comprises a TGFβ inhibitor and/or retinoic acid.
[15] The method of producing renal EPO-producing cells according to [13] or [14], wherein the renal interstitial progenitor cells are renal interstitial progenitor cells obtained by the method according to any one of [1] to [9].
[16] A method of producing renin-producing cells, the method comprising the step of culturing renal interstitial progenitor cells in a medium comprising a GSK-3β inhibitor and a TGFβ inhibitor, but not comprising an SHH signal activator, to produce the renin-producing cells.
[17] The method of producing renin-producing cells according to [16], wherein the renin-producing cells are mesangial cells or juxtaglomerular cells.
[18] The method of producing renin-producing cells according to [16] or [17], wherein the medium further comprises BMP, VEGF (vascular endothelial growth factor), and/or retinoic acid.
[19] The method of producing renin-producing cells according to any one of [16] to [18], wherein the medium further comprises a HIF inhibitor.
[20] The method of producing renin-producing cells according to any one of [16] to [19], wherein the renal interstitial progenitor cells are renal interstitial progenitor cells obtained by the method according to any one of [1] to [9].

### EFFECT OF THE INVENTION

According to a method of the invention, renal interstitial progenitor cells can be selectively obtained.

By further culturing the renal interstitial progenitor cells, production of renal EPO-producing cells, and renin-producing cells such as mesangial cells and juxtaglomerular cells is also possible.

The renal interstitial progenitor cells, the renal EPO-producing cells, and the renin-producing cells such as mesangial cells and juxtaglomerular cells obtained by the method of the present invention are useful as cell formulations for regenerative medicine applicable to chronic kidney diseases and the like. Further, by reconstruction of a kidney by mixing of the renal interstitial progenitor cells with nephron progenitor cells, a highly functional cell formulation for regenerative medicine can be obtained.

Further, the renal interstitial progenitor cells, the renal EPO-producing cells, and the renin-producing cells such as mesangial cells and juxtaglomerular cells obtained by methods of the present invention are also useful in the preparation of kidney disease models. The kidney disease models are useful also from the viewpoint of searching for therapeutic methods and therapeutic agents for kidney diseases.

### BRIEF DESCRIPTION OF THE DRAWINGS

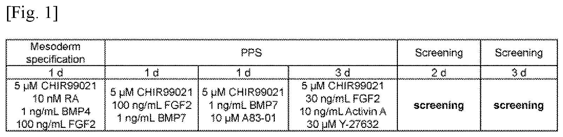

Fig. 1 is a diagram illustrating a protocol for differentiation from human iPS cells into the posterior primitive streak (PPS), and a strategy for screening for differentiation induction factors for differentiation from the obtained posterior primitive streak into renal interstitial progenitor cells.

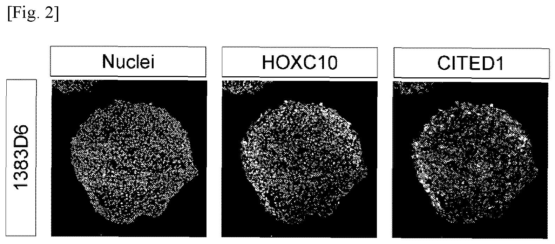

Fig. 2 is a diagram (photographs) showing results of immunohistochemical staining of PPS cells obtained by differentiation induction from human iPS cells.

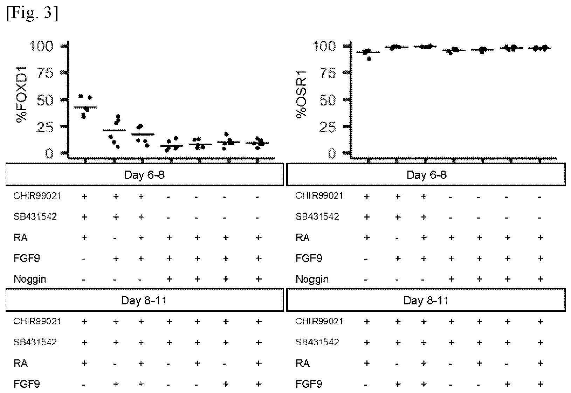

Fig. 3 is a diagram illustrating results of flow cytometric analysis (FOXD1 and OSR1), carried out on Day 11, of cells that were treated with various combinations of factors during the differentiation induction period of Days 6 to 11 (the period corresponding to "Screening" in Fig. 1). The following concentrations were employed: 1 µM CHIR99021 (CHIR), 10 µM SB431542 (SB), 100 nM retinoic acid (RA), 200 ng/mL FGF9, and 25 ng/mL Noggin.

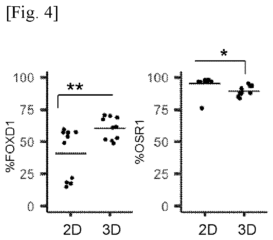

Fig. 4 is a diagram illustrating results of flow cytometric analysis (FOXD1 and OSR1), carried out on Day 11, of cells that were cultured by two-dimensional adherent culture (2D) or three-dimensional suspension culture (3D). The dots and the line in the center of each scatter diagram represent the experimental data and the mean, respectively. * indicates p < 0.05, and ** indicates p < 0.01 (Student's t-test).

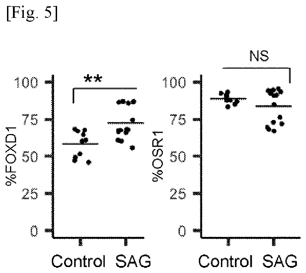

Fig. 5 is a diagram illustrating results of flow cytometric analysis (FOXD1 and OSR1), carried out on Day 11, of cells that were treated or not treated with Smoothened Agonist (SAG) (concentration, 500 nM) during the differentiation induction period of Days 6 to 8. ** indicates p < 0.01 (Student's t-test), and NS indicates "no significant difference".

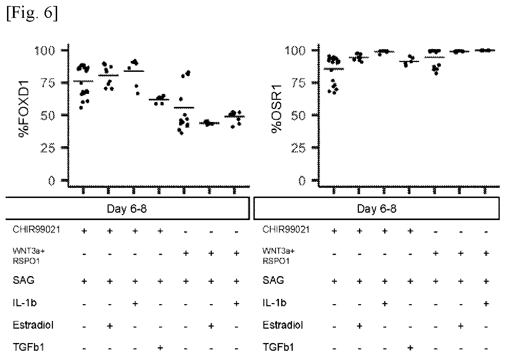

Fig. 6 is a diagram illustrating results of flow cytometric analysis (FOXD1 and OSR1), carried out on Day 11, of cells that were treated with various factors during the differentiation induction period of Days 6 to 8. The following concentrations were employed: 1 µM CHIR, 10% Wnt3a, 200 ng/mL RSPO1 (R-Spondin-1), 500 nM SAG, 10 ng/mL IL-1β, 50 ng/mL Estradiol, and 10 ng/mL TGFβ1.

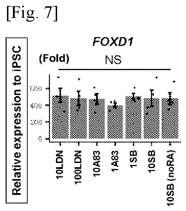

Fig. 7 is a diagram illustrating results of qRT-PCR (quantitative RT-PCR) analysis, carried out on Day 11, of FOXD1 after administration of various ALK inhibitors in combination with or not in combination with retinoic acid (RA) under SI+S induction conditions (Days 6 to 8: SAG + IL1β → Days 9 to 11: SAG). SB431542 was at 1 or 10 µM; A83-01 was at 1 or 10 µM; and LDN193189 was at 10 or 100 nM.

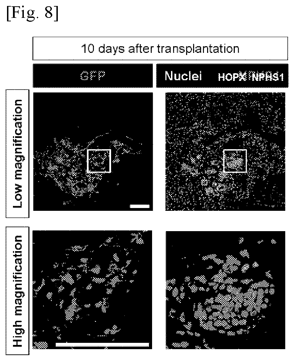

Fig. 8 is a diagram (photographs) showing immunostaining images for HOPX, NPHS1, and GFP on Day 10 after transplantation of a kidney graft prepared from hiPSC-derived GFP(+) NPCs and GFP(-) IPCs. The lower panels show magnified images of the boxed areas in the upper panels.

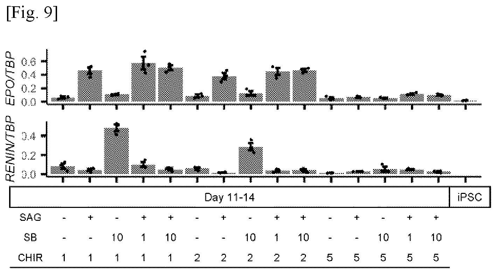

Fig. 9 is a diagram illustrating results of qRT-PCR analysis of EPO and renin carried out on Days 11 to 14, that is, after obtaining renal interstitial progenitor cells, for comparison of the effects of combinations of candidate differentiation induction factors for renal EPO-producing cells or renin-producing cells. The SAG concentration was 500 nM, and the concentrations of SB and CHIR are expressed in the unit of µM.

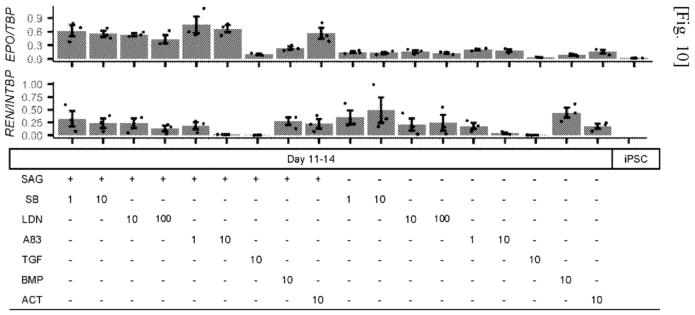

Fig. 10 is a diagram illustrating results of qRT-PCR analysis of EPO and renin on Days 11 to 14, that is, after obtaining renal interstitial progenitor cells, for comparison of the effects of combinations of various candidate differentiation induction factors. The SAG concentration was 500 nM; the concentrations of SB and A83 are expressed in the unit of µM; LDN is expressed in the unit of nM; and TGFβ1, BMP7, and activin (ACT) are expressed in the unit of ng/mL.

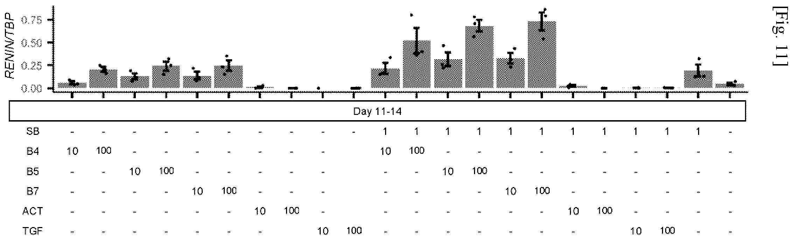

Fig. 11 is a diagram illustrating results of qRT-PCR analysis of renin on Days 11 to 14, that is, after obtaining renal interstitial progenitor cells, for comparison of the effects of various factors. In the presence of CHIR99021, RA, and FG-4592 (CFR), the effects of BMP4, BMP5, and BMP7 (10 ng/mL or 100 ng/mL) were investigated under conditions with or without treatment by SB431542 (1 µM). SB is expressed in the unit of µM, and TGFβ1 and ACT are expressed in the unit of ng/mL.

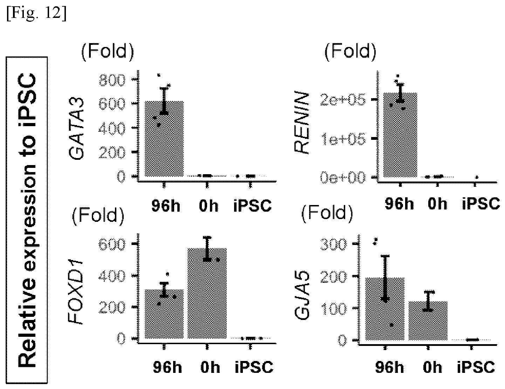

Fig. 12 is a diagram illustrating results of qRT-PCR analysis of marker expression for the mesangial cell lineage (GATA3, renin, and GJA5) and IPCs (FOXD1) at Hour 0 and Hour 96 after treatment with CFR + BMP7 + SB. iPSC represents the control.

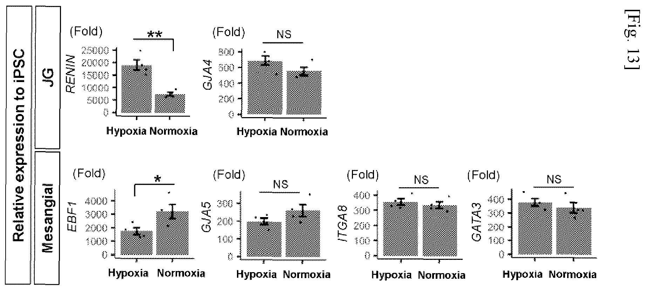

Fig. 13 is a diagram illustrating results of qRT-PCR analysis of cells that were subjected to treatment under hypoxic (CFR + B7 + SB in 5% O₂) or normal oxygen (CFR + B7 + SB minus FG-4592 in 20% O₂) conditions on Days 11 to 15, that is, after obtaining renal interstitial progenitor cells, which analysis was carried out for markers for JG (renin and GJA4) and mesangial cells (EBF1, GJA5, ITGA8, and GATA3). * indicates p < 0.05; ** indicates p < 0.01; and NS indicates "no significant difference" (Student's t-test).

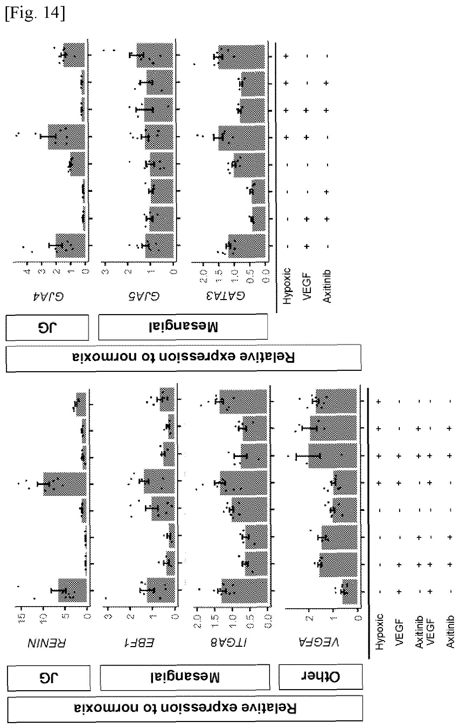

Fig. 14 is a diagram illustrating results of qRT-PCR analysis of cells that were subjected to treatment with VEGF (100 ng/mL) or axitinib (3 µM) and also to treatment under hypoxic conditions (CFR + B7 + SB in 5% O₂) or normal conditions (CFR + B7 + SB minus FG-4592 in 20% O₂) on Days 11 to 15, that is, after obtaining renal interstitial progenitor cells, which analysis was carried out for markers for JG (renin and GJA4), mesangial cells (EBF1, GJA5, ITGA8, and GATA3), and VEGFA.

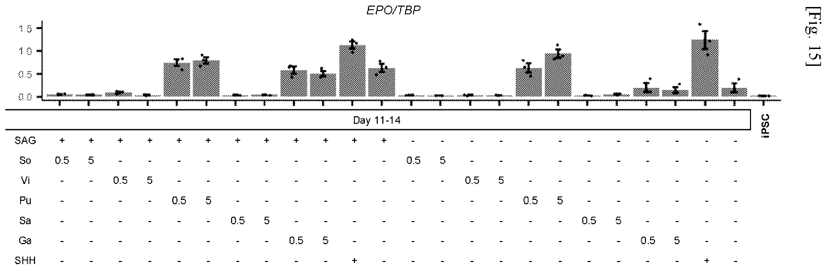

Fig. 15 is a diagram illustrating results of qRT-PCR analysis of EPO carried out on Days 11 to 14, that is, after obtaining renal interstitial progenitor cells, for comparison of the effects of various Sonic Hedgehog (SHH) signal modulators under induction conditions using CHIR99021, RA, and A83-01. So, Vi, Pu, Sa, and Ga represent sonidegib, vismodegib, purmophamine, SANT-1, and GANT61, respectively, and their concentrations are expressed in the unit of µM. The concentration of SAG was 500 nM, and the concentration of SHH was 100 ng/mL.

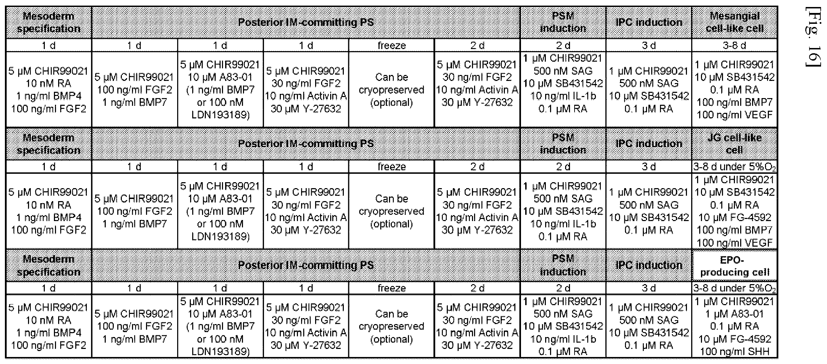

Fig. 16 is a diagram illustrating examples of protocols for allowing differentiation of pluripotent stem cells into mesangial cells, JG cells, or renal EPO-producing cells via IPCs.

### MODE FOR CARRYING OUT THE INVENTION

The present invention is described below in detail.

### <Renal Interstitial Progenitor Cells>

The renal interstitial progenitor cells are preferably derived from a mammal, more preferably derived from a primate or rodent, preferably derived from a human or mouse. Therefore, in the present description, the HOXC10-positive cells used to produce renal interstitial progenitor cells; and the renal EPO-producing cells, and the renin-producing cells such as mesangial cells and juxtaglomerular cells, obtained from renal interstitial progenitor cells; are also preferably derived from a mammal, more preferably derived from a primate or rodent, preferably derived from a human or mouse.

The renal interstitial progenitor cells are cells capable of differentiation in vitro into interstitial cells in the kidney, renal EPO-producing cells, and renin-producing cells such as mesangial cells and juxtaglomerular cells. The renal interstitial progenitor cells can be defined by FOXD1 (Forkhead Box D1) positivity. Further, the cells may be OSR1-positive. Further, the cells may be PDGFRB (platelet-derived growth factor receptor β)-positive. In the present description, positivity of a marker can be confirmed, for example, by immunostaining using a marker-specific antibody, or by RT-PCR using a marker-specific primer.

### <Method of Producing Renal Interstitial Progenitor Cells>

A method of producing renal interstitial progenitor cells according to one aspect of the present invention comprises the step of culturing HOXC10-positive cells in a medium comprising a GSK-3β inhibitor and a TGFβ inhibitor, to induce the renal interstitial progenitor cells (which may be hereinafter referred to as renal interstitial progenitor cell differentiation induction step).

Here, the medium preferably also comprises a SHH signal activator.

The medium preferably also contains IL-1β.

The medium preferably also comprises retinoic acid.

Examples of the medium include:
a medium comprising a GSK-3β inhibitor and a TGFβ inhibitor, and optionally comprising retinoic acid;
a medium comprising a GSK-3β inhibitor, a TGFβ inhibitor, and a SHH signal activator, and optionally comprising retinoic acid;
a medium comprising a GSK-3β inhibitor, a TGFβ inhibitor, and IL-1β, and optionally comprising retinoic acid; and
a medium comprising a GSK-3β inhibitor, a TGFβ inhibitor, a SHH signal activator, and IL-1β, and optionally comprising retinoic acid.

The HOXC10-positive cells are not limited as long as they are positive for these markers. The cells may be positive also for CITED1 (Cbp/P300 Interacting Transactivator With Glu/Asp Rich Carboxy-Terminal Domain 1). The cells may be positive also for HOXD11 and BRACHYURY (T). The HOXC10-positive cells include posterior primitive streak cells, neuromesodermal progenitor cells, and the posterior immature mesoderm.

The origin of the HOXC10-positive cells is not limited. The HOXC10-positive cells are preferably obtained by differentiation induction from pluripotent stem cells. The method of inducing differentiation from the pluripotent stem cells into the HOXC10-positive cells is not limited. For example, the later-mentioned method of differentiation induction from pluripotent stem cells into HOXC10-positive cells may be employed.

The HOXC10-positive cells subjected to the renal interstitial progenitor cell differentiation induction step may be a cell population containing another cell type, or may be purified HOXC10-positive cells. For example, a cell population containing HOXC10-positive cells at not less than 10%, not less than 20%, not less than 30%, not less than 40%, not less than 50%, 60%, 70%, or not less than 80% is subjected to the renal interstitial progenitor cell differentiation induction step.

The culture in the renal interstitial progenitor cell differentiation induction step may be carried out by either adherent culture or suspension culture. The culture is preferably carried out by suspension culture.

Here, the suspension culture means that cells are cultured in a state where they are not adhering to the culture substrate. This is carried out, for example, in a mode using a low-cell-adhesive culture vessel. Here, the adherent culture means that cells are cultured in a state where they are adhering to the culture substrate. This means, for example, culturing in a culture dish subjected to coating treatment. The coating agent is preferably an extracellular matrix, and examples of the extracellular matrix include substances such as collagen, proteoglycan, fibronectin, hyaluronic acid, tenascin, entactin, elastin, fibrillin, and laminin, and fragments thereof.

The medium used for the renal interstitial progenitor cell differentiation induction step can be prepared by adding a GSK-3β inhibitor and a TGFβ inhibitor, and when necessary, one or more of a SHH signal stimulant, IL-1β, and retinoic acid, to a basal medium for use in animal cell culture. Examples of the basal medium include IMDM, Medium 199, Eagle's Minimum Essential Medium (EMEM), αMEM, Dulbecco's modified Eagle's Medium (DMEM), Ham's F12 (F12) medium, RPMI 1640 medium, Fischer's medium, and mixed media thereof. The medium may contain serum (for example, fetal bovine serum (FBS)), or may be serum-free. When necessary, the medium may contain one or more of serum replacements such as albumin, transferrin, KnockOut Serum Replacement (KSR) (serum replacement for ES cell culture) (Invitrogen), N2 supplement (Invitrogen), B27 supplement (Invitrogen), fatty acids, insulin, collagen precursor, trace elements, 2-mercaptoethanol, and 3'-thiolglycerol, and may also contain one or more of substances such as lipids, amino acids, L-glutamine, GlutaMAX (Invitrogen), non-essential amino acids (NEAA), vitamins, growth factors, antibiotics, antioxidants, pyruvic acid, buffers, inorganic salts, and equivalents thereof. Media preliminarily optimized for stem cell culture, such as ReproFF2 (ReproCELL) and Stem Fit AK02N medium (Ajinomoto Healthy Supply Co., Inc.) may also be used.

### <GSK-3β Inhibitor>

The GSK-3β inhibitor is not limited as long as it is capable of inhibiting a function of GSK-3β such as kinase activity. Examples of the GSK-3β inhibitor include BIO (another name, GSK-3β inhibitor IX; 6-bromoindirubin-3'-oxime), which is an indirubin derivative; SB216763 (3-(2,4-dichlorophenyl)-4-(1-methyl-1*H*-indol-3-yl)-1*H*-pyrrole-2,5-dione), which is a maleimide derivative; GSK-3β inhibitor VII (α,4-dibromoacetophenone), which is a phenyl-α-bromomethyl ketone compound; L803-mts (another name, GSK-3β peptide inhibitor; Myr-N-GKEAPPAPPQSpP-NH₂), which is a cell membrane-permeable phosphorylated peptide; and CHIR99021, which has high selectivity (Nature (2008) 453: 519-523). These compounds are available from, for example, Stemgent, Calbiochem, and Biomol, or may be prepared by oneself. A preferred example of the GSK-3β inhibitor used in the present step is CHIR99021. The concentration of the GSK-3 β inhibitor in the medium can be appropriately selected by those skilled in the art depending on the GSK-3β inhibitor used, and is, for example, 0.1 µM to 10 µM, preferably 0.5 µM to 3 µM, more preferably 0.5 µM to 1.5 µM.

### <TGFβ Inhibitor>

The TGFβ inhibitor is a substance which inhibits signal transduction that proceeds from binding of TGFβ to its receptor, to SMAD. Examples of the TGFβ inhibitor include substances that inhibit binding of TGFβ to the ALK family, which is the receptor, and substances that inhibit phosphorylation of SMAD by the ALK family (also called ALK inhibitors). Examples of such substances include Lefty-1 (for example, NCBI accession numbers NM_010094 (mouse) and NM_020997 (human)); SB431542 and SB202190 (these are described in R. K. Lindemann et al., Mol. Cancer, 2003, 2:20); SB505124 (GlaxoSmithKline); NPC30345, SD093, SD908, and SD208 (Scios); LY2109761, LY364947, and LY580276 (Lilly Research Laboratories); A83-01 (WO 2009146408); LDN193189; and derivatives thereof. The TGFβ inhibitor may be preferably SB431542.

The concentration of the TGFβ inhibitor in the medium can be appropriately selected by those skilled in the art depending on the TGFβ inhibitor used. For example, SB431542 may be at 0.5 µM to 100 µM, preferably at 1 µM to 50 µM, more preferably at 5 µM to 25 µM. A83-01 may be at 0.1 µM to 100 µM, preferably at 0.5 µM to 50 µM, more preferably at 1 µM to 10 µM. LDN193189 may be at 0.01 µM to 1 µM, preferably at 0.05 µM to 0.5 µM, more preferably at 0.05 µM to 0.2 µM.

### <SHH Signal Activator>

The SHH (Sonic Hedgehog) signal stimulant is defined as a substance which causes disinhibition of Smoothened (Smo) caused by binding of SHH to its receptor Patched (Ptch1), and which also causes the subsequent activation of Gli2. Examples of the SHH signal stimulant include: proteins belonging to the Hedgehog family, such as SHH protein (for example, GenBank accession numbers NM_000193 and NP_000184) and IHH (Indian Hedgehog); SHH receptors; SHH receptor agonists; Hh-Ag1.5 (Li, X., et al., Nature Biotechnology, 23, 215-221 (2005)); Smoothened Agonist (SAG) (*N*-methyl-*N*'-(3 -pyridinylbenzyl)-*N*' -(3 -chlorobenzo[b]thiophene-2-carbonyl)-1,4-diaminocyclohexane; *N*-methyl-*N*'-(3-pyridinylbenzyl)-*N*'-(3-chlorobenzo[b]thiophene-2-carbonyl)-1,4-diaminocyclohexane); 20a-hydroxycholesterol; purmorphamine (PMA; 9-cyclohexyl-*N*-[4-(4-morpholinyl)phenyl]-2-(1-naphthalenyloxy)-9H-purine-6-amine); and derivatives thereof (Stanton BZ, Peng LF., Mol Biosyst. 6:44-54, 2010). One or more of these may be appropriately selected and used as the SHH signal activator. Preferred examples the SHH signal activator include SHH protein, purmorphamine, and SAG.

Regarding a preferred concentration in the medium, SAG is at 10 to 1000 nM, preferably at 100 to 1000 nM; SHH protein is at 10 to 1000 ng/mL, preferably at 50 to 200 ng/mL; purmophamine is preferably at 0.1 to 10 µM, preferably at 0.5 to 5 µM.

### <Retinoic Acid>

In the present description, the definition of retinoic acid includes not only retinoic acid itself, but also retinoic acid derivatives retaining the differentiation induction function of the naturally occurring retinoic acid. Examples of the retinoic acid derivatives include 3-dehydroretinoic acid; 4-[[(5,6,7,8-tetrahydro-5,5,8,8-tetramethyl-2-naphthalenyl)carbonyl]amino]-benzoic acid (AM580) (Tamura K, et al., Cell Differ. Dev. 32: 17-26 (1990)); 4-[(1*E*)-2-(5,6,7,8-tetrahydro-5,5,8,8-tetramethyl-2-naphthalenyl)-1-propen-1-yl]-benzoic acid (TTNPB) (Strickland S, et al., Cancer Res. 43: 5268-5272 (1983)); a compound described in Tanenaga, K. et al., Cancer Res. 40: 914-919 (1980); retinol palmitate; retinol; retinal; 3-dehydroretinol; and 3-dehydroretinal.

The concentration of retinoic acid in the medium is, for example, 1 nM to 1000 nM, preferably 10 nM to 500 nM, more preferably 50 nM to 250 nM.

### <IL-1β>

The IL-1β is not limited, and is preferably human IL-1β. Examples of the human IL-1β include a protein having the amino acid sequence of NCBI (National Center for Biotechnology Information) accession number 1TOO_A. The IL-1β also includes fragments and functionally modified products thereof as long as they have differentiation induction activity. As the IL-1(3, a commercially available product may be used, or a protein purified from cells or a protein produced by genetic recombination may be used. The concentration of the IL-1(3 in the medium is, for example, 1 ng/mL to 500 ng/mL, 1 ng/mL to 100 ng/mL, 5 ng/mL to 50 ng/mL, or 5 ng/mL to 25 ng/mL.

In a preferred mode of the method of producing renal interstitial progenitor cells of the present invention, the step of inducing renal interstitial progenitor cells from HOXC10-positive cells may be carried out by the following two stages:
1) the step of culturing the HOXC10-positive cells in a medium comprising a GSK-3β inhibitor, a TGFβ inhibitor, a SHH signal activator, and IL-1β, and optionally comprising retinoic acid; and
2) the step of culturing the cells obtained in Step 1) (posterior unsegmented mesodermal cells) in a medium comprising a GSK-3β inhibitor, a TGFβ inhibitor, and a SHH signal activator, and optionally comprising retinoic acid.

By performing the two stages in which IL-1β is removed in Step 2), the efficiency of differentiation induction into renal interstitial progenitor cells can be increased.

Examples of the number of days of culture in the renal interstitial progenitor cell differentiation induction step include not less than 2 days, not less than 3 days, not less than 4 days, and not less than 5 days. There is no upper limit of the number of days of the culture since long-term culture does not affect the production efficiency of the renal interstitial progenitor cells. It is, for example, not more than 30 days. In the renal interstitial progenitor cell differentiation induction step, the culture conditions are not limited as long as renal interstitial progenitor cells can be obtained. The culture temperature is about 30 to 40°C, preferably about 37°C. The oxygen concentration is a normal oxygen concentration (for example, 15 to 25%, preferably about 20%). The CO₂ concentration is preferably about 2 to 5%.

The renal interstitial progenitor cells obtained may be isolated or concentrated. The isolation or concentration can be carried out, for example, by flow cytometry using an antibody against PDGFRB.

By further culturing the renal interstitial progenitor cells obtained by the above method, renal EPO-producing cells, and renin-producing cells such as mesangial cells and juxtaglomerular cells can be obtained.

### <Method of Producing Renal EPO-Producing Cells>

One aspect of the present invention relates to a method of producing renal EPO-producing cells from renal interstitial progenitor cells.

More specifically, one aspect of the present invention provides a method of producing renal EPO-producing cells, the method comprising the step of culturing renal interstitial progenitor cells in a medium comprising a GSK-3β inhibitor, a SHH signal activator, and a HIF inhibitor, to produce the renal EPO-producing cells (which may be hereinafter referred to as "renal EPO-producing cell differentiation induction step").

The medium preferably also comprises a TGFβ inhibitor.

The medium preferably also comprises retinoic acid.

Examples of the medium include:
a medium comprising a GSK-3β inhibitor, a SHH signal activator, and a HIF inhibitor, and optionally comprising retinoic acid; and
a medium comprising a GSK-3β inhibitor, a SHH signal activator, a HIF inhibitor, and a TGFβ inhibitor, and optionally comprising retinoic acid.

The origin of the renal interstitial progenitor cells is not limited, and any renal interstitial progenitor cells may be used as long as they are FOXD1-positive cells. Preferably, renal interstitial progenitor cells obtained by the above-described method of producing renal interstitial progenitor cells may be used.

The renal EPO-producing cells are characterized by expression and/or production of EPO.

The culture in the renal EPO-producing cell differentiation induction step may be carried out by either adherent culture or suspension culture. The culture is preferably carried out by suspension culture.

The medium used for the renal EPO-producing cell differentiation induction step can be prepared by adding a GSK-3β inhibitor, a SHH signal activator, and a HIF inhibitor, and when necessary, a TGFβ inhibitor and/or retinoic acid, to a basal medium for use in animal cell culture. Examples of the basal medium include the media exemplified for the renal interstitial progenitor cell differentiation induction step.

As the GSK-3β inhibitor, the GSK-3β inhibitors (such as CHIR99021) described above for the renal interstitial progenitor cell differentiation induction step may be used. The same preferred concentration range may be used therefor.

As the SHH signal activator, the SHH signal activators described for the renal interstitial progenitor cell differentiation induction step (such as SAG, SHH protein, and purmorphamine) may be used. The same preferred concentration range may be used therefor.

The HIF (hypoxia-inducible factor) inhibitor is not limited as long as it is a substance that produces a function as a HIF inhibitor, and known HIF inhibitors may be used. Examples of the HIF inhibitor include *N*-[[4-hydroxy-2-oxo-1-(phenylmethyl)-1,2-dihydro-3-quinolinyl]carbonyl]glycine, *N*-[[1-(2-cyclopropylethyl)-6-fluoro-4-hydroxy-2-oxo-1,2-dihydro-3-quinolinyl]carbonyl]glycine, LW6, MK-8617, FG-2216, molidustat (BAY85-3934), KC7F2, roxadustat (FG-4592), and 2-methoxyestradiol.

The concentration of the HIF inhibitor is not limited as long as it is effective for the differentiation induction into renal EPO-producing cells. Examples of the concentration include 0.5 µM to 100µM, preferably 1 µM to 50 µM, more preferably 5 µM to 25 µM.

As the TGFβ inhibitor, the TGFβ inhibitors (such as SB431542, A83-01, and LDN193189) described above for the renal interstitial progenitor cell differentiation induction step may be used. The same preferred concentration range may be used therefor.

As described above for the renal interstitial progenitor cell differentiation induction step, the retinoic acid may also be its derivative. The same preferred concentration range may be used therefor.

The number of days of culture in the renal EPO-producing cell differentiation induction step is not limited as long as the period is sufficient for the production of the renal EPO-producing cells. Since long-term culture does not affect the production efficiency of the renal EPO-producing cells, there is no upper limit of the number of days of culture. Examples of the number of days of culture include not less than 2 days, not less than 3 days, not less than 4 days, and not less than 5 days. In the EPO-producing cell differentiation induction step, the culture temperature is about 30 to 40°C, preferably about 37°C, although the culture temperature is not limited thereto. In the renal EPO-producing cell differentiation induction step, the culture may be carried out at a normal oxygen concentration (such as 15 to 25%, preferably about 20%). The culture is preferably carried out under hypoxic conditions at an oxygen concentration of, for example, 1% to 10%, preferably 3% to 8%, more preferably 4% to 6%, especially preferably 5%. The CO₂ concentration is preferably about 2 to 5%.

### <Method of Producing Renin-Producing Cells>

One aspect of the present invention relates to a method of producing renin-producing cells from renal interstitial progenitor cells.

More specifically, one aspect of the present invention provides a method of producing renin-producing cells, the method comprising the step of culturing renal interstitial progenitor cells in a medium comprising a GSK-3β inhibitor and a TGFβ inhibitor, but not comprising a SHH signal activator, to produce the renin-producing cells (which may be hereinafter referred to as "renin-producing cell differentiation induction step").

The medium preferably also comprises BMP.

The medium preferably also comprises VEGF.

The medium preferably also comprises retinoic acid.

The medium preferably comprises a HIF inhibitor.

Examples of the medium include:
a medium comprising a GSK-3β inhibitor and a TGFβ inhibitor, not comprising a SHH signal activator, and optionally comprising retinoic acid;
a medium comprising a GSK-3β inhibitor, a TGFβ inhibitor, and BMP, not comprising a SHH signal activator, and optionally comprising retinoic acid;
a medium comprising a GSK-3β inhibitor, a TGFβ inhibitor, and VEGF, not comprising a SHH signal activator, and optionally comprising retinoic acid;
a medium comprising a GSK-3β inhibitor, a TGFβ inhibitor, and a HIF inhibitor, not comprising a SHH signal activator, and optionally comprising retinoic acid;
a medium comprising a GSK-3β inhibitor, a TGFβ inhibitor, BMP, and VEGF, not comprising a SHH signal activator, and optionally comprising retinoic acid;
a medium comprising a GSK-3β inhibitor, a TGFβ inhibitor, BMP, and a HIF inhibitor, not comprising a SHH signal activator, and optionally comprising retinoic acid;
a medium comprising a GSK-3β inhibitor, a TGFβ inhibitor, a HIF inhibitor, and VEGF, not comprising a SHH signal activator, and optionally comprising retinoic acid; and
a medium comprising a GSK-3β inhibitor, a TGFβ inhibitor, BMP, a HIF inhibitor, and VEGF, not comprising a SHH signal activator, and optionally comprising retinoic acid.

The origin of the renal interstitial progenitor cells used in the renin-producing cell differentiation induction step is not limited, and any renal interstitial progenitor cells may be used as long as they are FOXD1-positive cells. Preferably, renal interstitial progenitor cells obtained by the above-described method of producing renal interstitial progenitor cells may be used.

Examples of the renin-producing cells include mesangial cells and juxtaglomerular (JG) cells. Although both of these cells produce renin, juxtaglomerular cells show higher expression of renin. Mesangial cells express GJA5 (gap junction alpha-5) and EBF1 (EBF transcription factor 1). Juxtaglomerular cells express GJA4 (gap junction alpha-4).

The culture in the renin-producing cell differentiation induction step may be carried out by either adherent culture or suspension culture. The culture is preferably carried out by suspension culture.

The medium used for the renin-producing cell differentiation induction step can be prepared by adding a GSK-3β inhibitor and a TGFβ inhibitor, and when necessary, BMP, VEGF, a HIF inhibitor, retinoic acid and/or the like, to a basal medium for use in animal cell culture. Examples of the basal medium include the media exemplified for the renal interstitial progenitor cell differentiation induction step.

As the GSK-3β inhibitor, the GSK-3β inhibitors (such as CHIR99021) described above for the renal interstitial progenitor cell differentiation induction step may be used. The same preferred concentration range may be used therefor.

As the TGFβ inhibitor, the TGFβ inhibitors (such as SB431542, A83-01, and LDN193189) described above for the renal interstitial progenitor cell differentiation induction step may be used. The same preferred concentration range may be used therefor.

As described above for the renal interstitial progenitor cell differentiation induction step, the retinoic acid may also be its derivative. The same preferred concentration range may be used therefor.

Examples of the BMP include BMP4, BMP5, and BMP7. BMP7 is more preferred. The BMP is preferably human BMP. Examples of the human BMP7 include a protein having the amino acid sequence at 293 to 431 of NCBI (National Center for Biotechnology Information) accession number NP_001710.1. The BMP also includes fragments and functionally modified products thereof as long as they have an activity that induces differentiation into renin-producing cells. As the BMP, a commercially available product may be used, or a protein purified from cells or a protein produced by genetic recombination may be used. The concentration of the BMP used in this step is 1 ng/mL to 500 ng/mL, preferably 5 ng/mL to 200 ng/mL, more preferably 10 g/mL to 100 ng/mL.

The VEGF is not limited, and is preferably human VEGF. Examples of the human VEGF include a protein having the amino acid sequence of NCBI (National Center for Biotechnology Information) accession number AAK95847. The VEGF also includes fragments and functionally modified products thereof as long as they have an activity that induces differentiation into renin-producing cells. As the VEGF, a commercially available product may be used, or a protein purified from cells or a protein produced by genetic recombination may be used. The concentration of the VEGF used in this step is 1 ng/mL to 500 ng/mL, preferably 10 ng/mL to 200 ng/mL, more preferably 50 ng/mL to 150 ng/mL.

As the HIF inhibitor, the HIF inhibitors (such as FG-4592) described above for the renal EPO-producing cell differentiation induction step may be used. The same preferred concentration range may be used therefor.

The number of days of culture in the renin-producing cell differentiation induction step is not limited as long as the period is sufficient for the production of the renin-producing cells. Since long-term culture does not affect the production efficiency of the renin-producing cells, there is no upper limit of the number of days of culture. Examples of the number of days of culture include not less than 2 days, not less than 3 days, not less than 4 days, and not less than 5 days. The culture temperature is about 30 to 40°C, preferably about 37°C, and the CO₂ concentration is preferably about 2 to 5%, although the culture conditions in the renin-producing cell differentiation induction step are not limited thereto.

Although the oxygen concentration may be a normal oxygen concentration (for example, 15 to 25%, preferably about 20%), a low oxygen concentration (1% to 10%, preferably 3% to 8%, more preferably 4% to 6%, especially preferably 5%) is preferred. Under hypoxic culture conditions, the medium preferably comprises a HIF inhibitor.

As an example of the method of producing renin-producing cells, a preferred mode of a method of producing mesangial cells is described below.

### <Method of Producing Mesangial Cells>

One aspect of the present invention relates to a method of producing mesangial cells from renal interstitial progenitor cells.

More specifically, in one preferred mode, a method of producing mesangial cells, the method comprising the step of culturing renal interstitial progenitor cells in a medium comprising a GSK-3β inhibitor, a TGFβ inhibitor, BMP, and VEGF, but not comprising a SHH signal activator, to produce the mesangial cells (which may be hereinafter referred to as "mesangial cell differentiation induction step").

The medium preferably also comprises retinoic acid.

As described above, the origin of the renal interstitial progenitor cells used in the mesangial cell differentiation induction step is not limited, and any renal interstitial progenitor cells may be used. Preferably, renal interstitial progenitor cells obtained by the above-described method of producing renal interstitial progenitor cells may be used.

The culture in the mesangial cell differentiation induction step may be carried out by either adherent culture or suspension culture. The culture is preferably carried out by suspension culture.

The medium used for the mesangial cell differentiation induction step can be prepared by adding a GSK-3β inhibitor, a TGFβ inhibitor, BMP, and VEGF, and when necessary, retinoic acid, to a basal medium for use in animal cell culture. Examples of the basal medium include the media exemplified for the renal interstitial progenitor cell differentiation induction step.

The GSK-3β inhibitor, TGFβ inhibitor, retinoic acid, BMP, and VEGF are as described above, and their preferred substances and concentration ranges are also as described above.

The number of days of culture in the mesangial cell differentiation induction step is not limited as long as the period is sufficient for the production of the mesangial cells. Since long-term culture does not affect the production efficiency of the mesangial cells, there is no upper limit of the number of days of culture. Examples of the number of days of culture include not less than 2 days, not less than 3 days, not less than 4 days, and not less than 5 days. In the mesangial cell differentiation induction step, the culture temperature is about 30 to 40°C, preferably about 37°C; the oxygen concentration is a normal oxygen concentration (for example, 15 to 25%, preferably about 20%) or a low oxygen concentration (1% to 10%, preferably 3% to 8%, more preferably 4% to 6%, especially preferably 5%); and the CO₂ concentration is preferably about 2 to 5%; although the culture conditions are not limited thereto.

As an example of the method of producing renin-producing cells, a preferred mode of a method of producing juxtaglomerular cells is described below.

### <Method of Producing Juxtaglomerular Cells>

One aspect of the present invention relates to a method of producing juxtaglomerular (JG) cells from renal interstitial progenitor cells.

More specifically, in one preferred mode, a method of producing juxtaglomerular cells, the method comprising the step of culturing renal interstitial progenitor cells in a medium comprising a GSK-3β inhibitor, a TGFβ inhibitor, a HIF inhibitor, BMP, and VEGF, to produce the juxtaglomerular cells (which may be hereinafter referred to as "juxtaglomerular cell differentiation induction step").

The medium preferably also comprises retinoic acid.

As mentioned above, the origin of the renal interstitial progenitor cells used in the juxtaglomerular cell differentiation induction step is not limited, and any renal interstitial progenitor cells may be used. Preferably, renal interstitial progenitor cells obtained by the above-described method of producing renal interstitial progenitor cells may be used.

The culture in the juxtaglomerular cell differentiation induction step may be carried out by either adherent culture or suspension culture. The culture is preferably carried out by suspension culture.

The medium used for the juxtaglomerular cell differentiation induction step can be prepared by adding a GSK-3β inhibitor, a TGFβ inhibitor, a HIF inhibitor, BMP, and VEGF, and when necessary, retinoic acid, to a basal medium for use in animal cell culture. Examples of the basal medium include the media exemplified for the renal interstitial progenitor cell differentiation induction step.

The GSK-3β inhibitor, TGFβ inhibitor, HIF inhibitor, BMP, VEGF, and retinoic acid are as described above, and their preferred substances and concentration ranges are also as described above.

The number of days of culture in the juxtaglomerular cell differentiation induction step is not limited as long as the period is sufficient for the production of the juxtaglomerular cells. Since long-term culture does not affect the production efficiency of the juxtaglomerular cells, there is no upper limit of the number of days of culture. Examples of the number of days of culture include not less than 2 days, not less than 3 days, not less than 4 days, and not less than 5 days. In the juxtaglomerular cell differentiation induction step, the culture temperature is about 30 to 40°C, preferably about 37°C, although the culture temperature is not limited thereto. In the juxtaglomerular cell differentiation induction step, the culture may be carried out at a normal oxygen concentration. However, the culture is preferably carried out under hypoxic conditions at an oxygen concentration of, for example, 1% to 10%, preferably 3% to 8%, more preferably 4% to 6%, especially preferably 5%. The CO₂ concentration is preferably about 2 to 5%.

### <Differentiation Induction from Pluripotent Stem Cells into HOXC10-Positive Cells>

In one embodiment of the present invention, the HOXC10-positive cells are HOXC10-positive cells induced from pluripotent stem cells. By using HOXC10-positive cells induced from pluripotent stem cells, it is possible to obtain renal interstitial progenitor cells, and further, renal EPO-producing cells and renin-producing cells (such as mesangial cells and juxtaglomerular cells), from the pluripotent stem cells.

Pluripotent stem cells are stem cells having pluripotency that allows differentiation into many kinds of cells present in a living body, which stem cells also have the growth ability. The pluripotent stem cells include arbitrary cells which can be induced into renal interstitial progenitor cells and renal erythropoietin-producing cells, and renin-producing cells. Examples of the pluripotent stem cells include, but are not limited to, embryonic stem (ES) cells, embryonic stem cells derived from a cloned embryo obtained by nuclear transfer (ntES cells), germline stem cells ("GS cells"), embryonic germ cells ("EG cells"), induced pluripotent stem (iPS) cells, and pluripotent cells derived from cultured fibroblasts or bone marrow stem cells (Muse cells). The pluripotent stem cells are preferably iPS cells from the viewpoint of the fact that these cells can be obtained without destroying embryos, eggs, or the like during the production process.

Methods for producing iPS cells are known in the art. These cells can be produced by introducing reprogramming factors into arbitrary somatic cells. Examples of the reprogramming factors herein include genes such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1, and gene products thereof. These reprogramming factors may be used either individually or in combination. Examples of the combinations of the reprogramming factors include those described in WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26: 2467-2474; Huangfu D, et al. (2008), Nat. Biotechnol. 26: 1269-1275; Shi Y, et al. (2008), Cell Stem Cell, 3, 568-574; Zhao Y, et al. (2008), Cell Stem Cell, 3: 475-479; Marson A, (2008), Cell Stem Cell, 3, 132-135; Feng B, et al. (2009), Nat. Cell Biol. 11: 197-203; R. L. Judson et al., (2009), Nat. Biotechnol., 27: 459-461; Lyssiotis CA, et al. (2009), Proc Natl Acad Sci U S A. 106: 8912-8917; Kim JB, et al. (2009), Nature. 461: 649-643; Ichida JK, et al. (2009), Cell Stem Cell. 5: 491-503; Heng JC, et al. (2010), Cell Stem Cell. 6: 167-74; Han J, et al. (2010), Nature. 463: 1096-100; Mali P, et al. (2010), Stem Cells. 28: 713-720; and Maekawa M, et al. (2011), Nature. 474: 225-9.

Examples of the somatic cells include, but are not limited to, any of fetal somatic cells, neonatal somatic cells, and mature, healthy or diseased somatic cells, as well as any of primary cultured cells, subcultured cells, and established cell lines. Specific examples of the somatic cells include: (1) tissue stem cells (somatic stem cells) such as neural stem cells, hematopoietic stem cells, mesenchymal stem cells, and dental pulp stem cells; (2) tissue progenitor cells; and (3) differentiated cells such as blood cells (peripheral blood cells, cord blood cells, and the like), lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells, and adipocytes.

In cases where iPS cells are used as a material of cells to be transplanted, it is preferred to use somatic cells whose HLA genotype is the same or substantially the same as that of the individual to which the cells are to be transplanted, from the viewpoint of preventing the rejection reaction. The term "substantially the same" herein means that the HLA genotype is matching to an extent at which the immune reaction against the transplanted cells can be suppressed with an immunosuppressant. For example, the somatic cells may have matched HLA types at the three loci HLA-A, HLA-B, and HLA-DR, or at the four loci further including HLA-C.

For the differentiation induction from the pluripotent stem cells into the HOXC10-positive cells, for example, a method including the following steps may be used:
(i) the step of culturing the pluripotent stem cells in a medium comprising bFGF, BMP (bone morphogenetic protein) 4, a GSK-3β inhibitor, and retinoic acid;
(ii) the step of culturing the cells obtained in Step (i), in a medium comprising bFGF, a GSK-3β inhibitor, and BMP7;
(iii) the step of culturing the cells obtained in Step (ii), in a medium comprising a GSK-3β inhibitor and a TGFβ inhibitor; and
(iv) the step of culturing the cells obtained in Step (iii), in a medium comprising bFGF, a GSK-3β inhibitor, activin, and a ROCK inhibitor.

In the middle or at the end of Step (iv), the cells can be cryopreserved. After thawing, the cells can be subjected to differentiation induction into renal interstitial progenitor cells or the like.

Each step is further described below.

### (i) Step of Culturing Pluripotent Stem Cells in Medium Comprising bFGF, BMP4, GSK-3β Inhibitor, and Retinoic Acid or Derivative Thereof

In Step (i), pluripotent stem cells are separated by an arbitrary method known in the art, and cultured preferably by adherent culture.

Examples of the method of separating the pluripotent stem cells include mechanical separation; and separation using a separation solution having protease activity and collagenase activity (for example, Accutase (TM) or Accumax (TM) (Innovative Cell Technologies, Inc.)), or a separation solution having only collagenase activity. A mixture of TrypLE Select Enzyme (Thermo Fisher Scientific) and EDTA/PBS may also be used. As the human pluripotent stem cells used in Step (i), colonies cultured to 70% to 80% confluence with respect to the dish used are preferably used.

The medium used in Step (i) can be prepared by adding bFGF, BMP4, a GSK-3β inhibitor, and retinoic acid or a derivative thereof to a basal medium for use in animal cell culture. Examples of the basal medium include the media exemplified for the renal interstitial progenitor cell differentiation induction step.

As the GSK-3β inhibitor used in Step (i), the GSK-3β inhibitors (such as CHIR99021) described above for the renal interstitial progenitor cell differentiation induction step may be used. The concentration can be adjusted within the same concentration range.

The bFGF (basic FGF) used in Step (i) is preferably human bFGF, and examples of the human bFGF include a protein having the amino acid sequence of NCBI (National Center for Biotechnology Information) accession number ABO43041.1. The bFGF also includes fragments and functionally modified products thereof as long as they have differentiation induction activity. As the bFGF, a commercially available product may be used, or a protein purified from cells or a protein produced by genetic recombination may be used. The concentration of the bFGF used in this step is 1 ng/mL to 1000 ng/mL, preferably 10 ng/mL to 500 ng/mL, more preferably 50 ng/mL to 250 ng/mL.

The BMP4 used in Step (i) is preferably human BMP4, and examples of the human BMP4 include a protein having the amino acid sequence of NCBI (National Center for Biotechnology Information) accession number AAH20546.1. The BMP4 also includes fragments and functionally modified products thereof as long as they have differentiation induction activity. As the BMP4, a commercially available product may be used, or a protein purified from cells or a protein produced by genetic recombination may be used. The concentration of the BMP4 used in this step is 0.1 ng/mL to 100 ng/mL, preferably 0.5 ng/mL to 50 ng/mL, more preferably 0.5 ng/mL to 5 ng/mL.

As described above for the renal interstitial progenitor cell differentiation induction step, the retinoic acid used in Step (i) may also be its derivative, whose preferred concentration range is 1 nM to 50 nM.

In Step (i), the culture temperature is about 30 to 40°C, preferably about 37°C; the oxygen concentration is a normal oxygen concentration (for example, 15 to 25%, preferably about 20%); and the CO₂ concentration is preferably about 2 to 5%; although the culture conditions are not limited thereto. The culture period in Step (i) is, for example, 1 to 2 days, preferably 1 day.

### (ii) Step of Culturing Cells Obtained in Step (i), in Medium Comprising bFGF, GSK-3β Inhibitor, and BMP7

In Step (ii), a cell population obtained in the Step (i) may be isolated and subjected to adherent culture in a separately provided culture dish that has been subjected to coating treatment. Alternatively, the cells obtained by adherent culture in Step (i) may be continuously cultured as they are by replacement of the medium.

The medium used in Step (ii) can be prepared by adding bFGF, a GSK-3β inhibitor, and BMP7 to a basal medium for use in animal cell culture. Examples of the basal medium include the media exemplified for the renal interstitial progenitor cell differentiation induction step.

The bFGF used in Step (ii) and its preferred concentration range are as described in Step (i).

As the GSK-3β inhibitor used in Step (ii), the GSK-3β inhibitors (such as CHIR99021) described above for the renal interstitial progenitor cell differentiation induction step may be used. The concentration can be adjusted within the same concentration range.

The BMP7 used in Step (ii) is preferably human BMP7, and examples of the human BMP7 include a protein having the amino acid sequence of NCBI (National Center for Biotechnology Information) accession number NM_001719.2. The BMP7 also includes fragments and functionally modified products thereof as long as they have differentiation induction activity. As the BMP7, a commercially available product may be used, or a protein purified from cells or a protein produced by genetic recombination may be used. The concentration of the BMP7 used in this step is 0.1 ng/mL to 100 ng/mL, preferably 0.5 ng/mL to 50 ng/mL, more preferably 0.5 ng/mL to 5 ng/mL.

In Step (ii), the culture temperature is about 30 to 40°C, preferably about 37°C; the oxygen concentration is a normal oxygen concentration (for example, 15 to 25%, preferably about 20%); and the CO₂ concentration is preferably about 2 to 5%; although the culture conditions are not limited thereto. The culture period in Step (ii) is, for example, 10 hours to 2 days, or 1 to 2 days, preferably 0.5 to 1 day.

### (iii) Step of Culturing Cells Obtained in Step (ii), in Medium Comprising GSK-3β Inhibitor and TGFB Inhibitor

In Step (iii), a cell population obtained in the Step (ii) may be isolated and subjected to adherent culture in a separately provided culture dish that has been subjected to coating treatment. Alternatively, the cells obtained by adherent culture in Step (ii) may be continuously cultured as they are by replacement of the medium.

The medium used in Step (iii) can be prepared by adding a GSK-3β inhibitor and a TGFβ inhibitor to a basal medium for use in animal cell culture. The medium may also contain BMP7. Examples of the basal medium include the media exemplified for the renal interstitial progenitor cell differentiation induction step.

As the GSK-3β inhibitor used in Step (iii), the GSK-3β inhibitors (such as CHIR99021) described above for the renal interstitial progenitor cell differentiation induction step may be used. The concentration can be adjusted within the same concentration range. In cases where BMP7 is added, the BMP7 is the same as in Step (ii), and its preferred concentration range is also the same.

As the TGFβ inhibitor used in Step (iii), one or more of the TGFβ inhibitors (such as SB431542, A83-01, and LDN193189) described above for the renal interstitial progenitor cell differentiation induction step may be used. Their preferred concentration ranges are as described above.

In Step (iii), the culture temperature is about 30 to 40°C, preferably about 37°C; the oxygen concentration is a normal oxygen concentration (for example, 15 to 25%, preferably about 20%); and the CO₂ concentration is preferably about 2 to 5%; although the culture conditions are not limited thereto. The culture period in Step (iii) is, for example, 0.5 to 3 days, preferably about 1 day.

### (iv) Step of Culturing Cells Obtained in Step (iii), in Medium Comprising bFGF, GSK-3β Inhibitor, Activin, and ROCK Inhibitor

By this step, HOXC10-positive posterior primitive streak cells (metanephric-lineage late primitive streak) are induced. In the middle or at the end of Step (iv), the cells can be cryopreserved. After thawing, the cells can be subjected to differentiation induction into renal interstitial progenitor cells or the like.

In Step (iv), a cell population obtained in the Step (iii) may be isolated and subjected to adherent culture in a separately provided culture dish that has been subjected to coating treatment. Alternatively, the cells obtained by adherent culture in Step (iii) may be continuously cultured as they are by replacement of the medium.

The medium used in Step (iv) can be prepared by adding bFGF, a GSK-3β inhibitor, activin, and a ROCK inhibitor to a basal medium for use in animal cell culture. Examples of the basal medium include the media exemplified for the renal interstitial progenitor cell differentiation induction step.

As the GSK-3β inhibitor used in Step (iv), the GSK-3β inhibitors (such as CHIR99021) described above for the renal interstitial progenitor cell differentiation induction step may be used. The concentration can be adjusted within the same concentration range. The bFGF and its preferred concentration range are the same as those in Step (i).

The activin used in Step (iv) includes activins derived from human and other animals, and functionally modified products thereof. For example, commercially available products from R&D Systems and the like may be used. The concentration of the activin used in Step (iv) is 1 ng/mL to 100 ng/mL, preferably 5 ng/mL to 50 ng/mL, more preferably 5 ng/mL to 25 ng/mL.

The ROCK inhibitor used in Step (iv) is not limited as long as the function of Rho-kinase (ROCK) can be suppressed therewith. Examples of the ROCK inhibitor include Y-27632 (see, for example, Ishizaki et al., Mol. Pharmacol. 57, 976-983 (2000), and Narumiya et al., Methods Enzymol. 325, 273-284 (2000)), Fasudil/HA1077 (see, for example, Uenata et al., Nature 389: 990-994 (1997)), H-1152 (see, for example, Sasaki et al., Pharmacol. Ther. 93: 225-232 (2002)), Wf-536 (see, for example, Nakajima et al., Cancer Chemother Pharmacol. 52(4): 319-324 (2003)), and derivatives thereof; and antisense nucleic acids, RNA interference-inducing nucleic acids (for example, siRNAs), and dominant negative mutants against ROCK, and expression vectors therefor. As the ROCK inhibitor, other known low molecular weight compounds may also be used (see, for example, US 2005/0209261 A, US 2005/0192304 A, US 2004/0014755 A, US 2004/0002508 A, US 2004/0002507 A, US 2003/0125344 A, US 2003/0087919 A, WO 2003/062227, WO 2003/059913, WO 2003/062225, WO 2002/076976, and WO 2004/039796). One or more ROCK inhibitors may be used. A preferred example of the ROCK inhibitor is Y-27632. The concentration of the ROCK inhibitor used in Step (iv) can be appropriately selected by those skilled in the art depending on the ROCK inhibitor used, and the concentration is, for example, 0.1 µM to 100 µM, preferably 1 µM to 75 µM, more preferably 5 µM to 50 µM.

In Step (iv), the culture temperature is about 30 to 40°C, preferably about 37°C; the oxygen concentration is a normal oxygen concentration (for example, 15 to 25%, preferably about 20%); and the CO₂ concentration is preferably about 2 to 5%; although the culture conditions are not limited thereto. The culture period in Step (iv) is not limited as long as it is a period sufficient for differentiation induction into the HOXC10-positive posterior primitive streak cells. The culture is carried out for, for example, 1 to 5 days, preferably 3 days.

### <Pharmaceutical Composition Comprising Renal Interstitial Progenitor Cells, Etc.>

The present invention also provides a cell formulation comprising renal interstitial progenitor cells, renal EPO-producing cells, or renin-producing cells (mesangial cells, juxtaglomerular cells, or the like) obtained by the above method, or a combination of a plurality of kinds of these cells. Such a cell formulation can be used as a pharmaceutical composition for treatment of a kidney disease or the like.

Further, by mixing the renal interstitial progenitor cells with nephron progenitor cells (OSR1-positive, preferably also HOXD11-, WT1-, SIX2-, and SALL1-positive cells) such as those disclosed in Patent Document 2, a cell formulation capable of reconstruction of a kidney tissue can be provided. The ratio between the renal interstitial progenitor cells and the nephron progenitor cells is preferably within the range of 1:2 to 2:1, more preferably about 1:1. Such a cell formulation may also contain renal EPO-producing cells or renin-producing cells (mesangial cells, juxtaglomerular cells, or the like).

After mixing the renal interstitial progenitor cells and the nephron progenitor cells, a renal organoid may be formed.

The kidney organoid can be obtained by, for example, culturing renal interstitial progenitor cells obtained by the above method and nephron progenitor cells, to prepare a cell cluster, and then co-culturing the cell cluster with feeder cells such as 3T3-Wnt4 cells, with fetal mouse spinal cord cells, or with fetal mouse kidney cells, or subjecting the cell cluster to semi-gas phase culture using a basal medium comprising a GSK-3β inhibitor.

Examples of the method of administering the cell formulation to a patient requiring the treatment include: a method in which the cells are formed into a sheet, and the sheet is attached to a kidney of the patient; a method in which the cells are suspended in physiological saline or the like to prepare a cell suspension, or the cells are subjected to three-dimensional culture to obtain a cell cluster, followed by directly transplanting the cell suspension or the cell cluster to a kidney of the patient; and a method in which three-dimensional culture is carried out on a scaffold constituted by Matrigel or the like, and the resulting cell cluster is transplanted. The site of transplantation is not limited as long as it is in a kidney. The site is preferably under the renal capsule. Examples of the kidney disease include acute renal damage, chronic renal failure, and chronic kidney diseases that have not progressed to chronic renal failure.

The number of the renal interstitial progenitor cells contained in the cell formulation is not limited as long as the graft can survive after the administration. The cell formulation may be prepared to contain an increased or decreased number of cells depending on the size of the affected area and the size of the body.

The renal interstitial progenitor cells, renal EPO-producing cells, and renin-producing cells (mesangial cells, juxtaglomerular cells, and the like) obtained by the above method can be used also for cleaning and evaluation of candidate substances for therapeutic agents for kidney diseases, hypertension, and the like.

### EXAMPLES

The present invention is concretely described below based on Examples. However, the present invention is not limited to the following modes.

### <Induction of Posterior Primitive Streak (PPS)>

Human iPS cells (hiPSCs) (17K6:OSR1-GFP/FOXD1-tdTomato double knock-in hiPSCs) were first subjected to enzyme treatment for 5 minutes using a 1:1 mixture of TrypLE Select Enzyme (Thermo Fisher Scientific) and 0.5 mM EDTA/PBS, and then washed with PBS(-). Thereafter, the cells were detached using a cell scraper, and then dissociated into single cells by gentle pipetting. One day before the start of differentiation, the cells were plated into a 24-well plate (CORNING) at a density of 1.3 × 10⁴ cells/cm² together with 0.5 mL of Stem Fit AK02N (Ajinomoto Healthy Supply Co., Inc.) supplemented with 10 µM Y-27632 and 2.4 µL/mL iMatrix-511 (Matrixome). Twenty-four hours later (Day 0), 5 µM CHIR99021 (Wako), 10 nM RA (Sigma), 1 ng/mL BMP4 (Peprotech), and 100 ng/mL bFGF (Wako) were added to a serum-free differentiation medium comprising DMEM/F12 Glutamax (Thermo Fisher Scientific), B27 supplement minus vitamin A (Thermo Fisher Scientific), and 0.5 × penicillin/streptomycin (hereinafter referred to as basal medium), and then culture was started. Twenty-four hours thereafter (Day 1), culture was performed with a basal medium supplemented with 5 µM CHIR99021, 100 ng/mL bFGF, and 1 ng/mL BMP7 (R&D Systems). On Day 2, the medium was switched to a basal medium supplemented with 5 µM CHIR99021, 1 ng/mL BMP7, and 10 µM A83-01 (Wako). On Day 3, the medium was switched to a basal medium supplemented with 5 µM CHIR99021, 30 ng/mL bFGF, 10 ng/mL activin A (R&D Systems), and 30 µM Y-27632 (Wako). On Day 4, the cells were washed with PBS(-), treated with Accumax (Innovative Cell Technologies), and then dissociated into single cells by gentle pipetting, followed by plating into a 24-well plate (CORNING) at a density of 1.05 × 10⁵ cells/cm² (2D culture) or plating into a 96-well low-cell-adhesive plate (Thermo Fisher Scientific) at a density of 2.0 × 10⁴ cells/cm² (3D culture). Culture was performed for 48 hours using a basal medium supplemented with 5 µM CHIR99021, 30 ng/mL bFGF, 10 ng/mL activin A, and 30 µM Y-27632 (in addition, 1.2 µL/well iMatrix-511 or a laminin-E8 fragment library in the case of 2D culture). By this, HOXC10- and CITED1-positive PPS cells were induced.

Fig. 2 shows the result of immunohistochemical staining of the PPS cells obtained by the differentiation induction from the human iPS cells.

### <Induction of Renal Interstitial Progenitor (IPC) Cells>

For the PPS cells on Day 6 of the culture protocol in Fig. 1, several differentiation induction conditions were tested by combining various factors. As a result, FOXD1-positive cells could be induced by 5 days of treatment using the combination of the GSK-3β inhibitor CHIR99021, the ALK inhibitor SB431542, and retinoic acid (RA) (Fig. 3). Based on comparison between the induced IPC cells and the PPS cells using RNA-seq, the IPC cells on Day 11 showed increased expression of IPC marker genes and down-regulation of primitive streak marker genes.

When CHIR99021 + SB431542 + RA were used as the IPC differentiation induction conditions, and the culture on Days 4 to 11 was performed by suspension culture (3D) in the differentiation induction protocol in Fig. 1, a higher induction efficiency for FOXD1-positive cells was found compared to the adherent (2D) culture (Fig. 4).

The induction efficiency for FOXD1-positive cells was found to be further increased by addition of Smoothened (SMO) Agonist (SAG), which is an activator of the Hedgehog pathway, on Days 6 to 8 (Fig. 5).

As a result of IPA analysis (QIAGEN), increased expression in terms of the WNT/Ca²⁺ pathway, the estrogen receptor signal, liver fibrosis, and the IL-1 signal was suggested. In view of this, a test was carried out by adding each of WNT3a plus RSPO1, β-estradiol, TGFβ1, and IL-1β on Days 6 to 8 and Days 8 to 11. As a result, it could be confirmed that the induction efficiency for OSR1-positive FOXD1-positive cells increases by addition of IL-1β and SAG on Days 6 to 8 (Fig. 6).

Further, it could be confirmed that use of various ALK inhibitors instead of SB431542 on Days 6 to 11 similarly allows induction of an IPC marker (Fig. 7).

### <Transplantation of IPCs and NPCs>

Nephron progenitor cells (NPCs) were induced by differentiation from iPS cells according to the method described in Patent Document 2.

The IPCs on Day 11 of differentiation induction, obtained as described above, and NPC aggregates were incubated in Accumax (Innovative Cell Technologies) at 37°C for 5 minutes, and then dissociated into single cells by gentle pipetting. The dissociated cells were mixed, and resuspended in a basal medium supplemented with 10 µM Y-27632, 10 ng/mL bFGF, 1 µM CHIR99021, and 0.1 µM RA, followed by plating the cells in a 96-well low-cell-adhesive U-bottom plate at densities of 5.0 × 10⁴ NPCs/well and 5.0 × 10⁴ IPCs/well to allow formation of aggregates. Twenty-four hours later, the mixed renal progenitor cell aggregates were collected and transplanted.

After transplanting the mixed renal progenitor cell aggregates under the renal capsule of an immunodeficient mouse (NSG mouse), 20 µL of Matrigel was inserted to release the tension, to secure a space for the aggregates.

Ten days after the transplantation, analysis was carried out by immunostaining. As a result, it was shown that glomeruli derived from hiPSCs containing NPHS1(+) podocytes were integrated with mouse blood vessels, and that a HOPX(+) mesangium-like structure was contained therein (Fig. 8).

### <Further Differentiation Induction from IPCs>

Subsequently, a study was carried out to investigate whether or not the induced IPCs can differentiate into renin-producing cells or renal EPO-producing cells in vitro.

First, using the IPCs on Day 11, culture was performed under 5% O₂ hypoxic conditions in the presence of FG-4592 (*N*-[(4-hydroxy-1-methyl-7-phenoxy-3-isoquinolinyl)carbonyl]-glycine) and RA with addition of various factors. By measuring the expression of EPO and renin, screening for induction factors for renin-producing cells and renal EPO-producing cells was carried out.

As a result, the combination of CHIR99021 and SB431542 was found to increase the expression of renin in the absence of SAG, and the combination of SB431542 and SAG was found to increase the expression of EPO (Fig. 9).

Further, involvement of BMP was investigated. As a result of evaluation with addition of various factors to a medium comprising CHIR99021, FG-4592, and RA (CFR), BMP7 positively affected the expression of renin in the absence of SAG, and the combination of A83-01 and SAG caused higher EPO expression compared to the combination of SB431542 and SAG (Fig. 10).

Further, as a result of a study of the effect of addition of BMP4, BMP5, or BMP7 to a medium comprising CHIR99021, FG-4592, and RA (CFR) with or without addition of SB431542, all of the three BMPs were found to cause high expression of renin in the presence of SB431542 (Fig. 11).

Further, as a result of 96 hours of treatment in CFR medium supplemented with BMP7 and SB431542 (CFR + B7 + SB medium), increased expression of GATA3, renin, and GJA5, and decreased expression of FOXD1 were found (Fig. 12).

Subsequently, a differentiation induction test was carried out under normal oxygen concentration conditions after exclusion of FG-4592 from CFR + B7 + SB medium at 20% oxygen concentration. As a result, the expression of EBF1, which is an important marker for the late mesangium development, was increased. On the other hand, under hypoxic conditions using 5% O₂ CFR + B7 + SB medium, the expression of renin was significantly induced (Fig. 13).

Further, the effect of VEGF was studied. As a result, it was found that addition of VEGF increases the expression of markers for juxtaglomerular (JG) cells and mesangial cells, but that the effect is reduced by axitinib (VEGF receptor inhibitor) (Fig. 14).

Subsequently, the effects of various SHH signal modulators in a system for differentiation induction from IPCs to EPO-producing cells were investigated (Fig. 15). Several SMO antagonists (sonidegib, vismodegib, and SANT-1) and agonists (SAG and purmophamine), a GLI1 inhibitor (GANT61), and human SHH protein were tested. As a result, although both the SMO agonists and human SHH protein caused up-regulation of EPO, human SHH protein alone caused the highest expression, and, while all SMO antagonists reduced the effect of SAG, the GLI1 inhibitor did not reduce the effect of SAG.

As a result of qRT-PCR, the EPO expression was found to have reached the peak around Hour 96. Further, production of EPO-positive cells was confirmed according to immunostaining analysis. These results suggest that the IPCs induced by the present inventors differentiate into EPO-producing cells by SHH activation.

As described above, an induction system that induces hiPSC-derived IPCs into mesangial cells, JG cells, and renal EPO-producing cells separately was successfully developed. Preferred differentiation protocols are illustrated in Fig. 16.

## Claims

1. A method of producing renal interstitial progenitor cells, the method comprising the step of culturing HOXC10-positive cells in a medium comprising a GSK (glycogen synthase kinase)-3β inhibitor, a SHH (Sonic Hedgehog) signal activator, and a TGF (transforming growth factor) β inhibitor, to induce the renal interstitial progenitor cells.

2. The method of producing renal interstitial progenitor cells according to claim 1, wherein the medium further comprises IL (interleukin)-1β.

3. The method of producing renal interstitial progenitor cells according to claim 2, further comprising the following steps 1) and 2):
1) the step of culturing HOXC10-positive cells in a medium comprising a GSK-3β inhibitor, a SHH signal activator, a TGFβ inhibitor, and IL-1β; and
2) the step of subsequently performing culture in a medium comprising a GSK-3β inhibitor, a SHH signal activator, and a TGFβ inhibitor.

4. The method of producing renal interstitial progenitor cells according to any one of claims 1 to 3, wherein the medium further comprises retinoic acid.

5. The method of producing renal interstitial progenitor cells according to any one of claims 1 to 4, wherein the culture is carried out by suspension culture.

6. The method of producing renal interstitial progenitor cells according to any one of claims 1 to 5, wherein the GSK-3β inhibitor is CHIR99021; the SHH signal activator is SHH protein, Purmorphamine, or SAG (Smoothened Agonist); and the TGFβ inhibitor is SB431542, A83-01, or LDN193189.

7. The method of producing renal interstitial progenitor cells according to any one of claims 1 to 6, wherein the HOXC10-positive cells are HOXC10-positive cells induced by differentiation from pluripotent stem cells.

8. The method according to claim 7, wherein the HOXC10-positive cells are HOXC10-positive cells produced by a method comprising the following steps (i) to (iv):
(i) the step of culturing the pluripotent stem cells in a medium comprising bFGF (basic fibroblast growth factor), BMP (bone morphogenetic protein) 4, a GSK-3β inhibitor, and retinoic acid or a derivative thereof;
(ii) the step of culturing the cells obtained in Step (i), in a medium comprising bFGF, a GSK-3β inhibitor, and BMP7;
(iii) the step of culturing the cells obtained in Step (ii) in a medium comprising a GSK-3β inhibitor and a TGFβ inhibitor; and
(iv) the step of culturing the cells obtained in Step (iii), in a medium comprising bFGF, a GSK-3β inhibitor, activin, and a ROCK inhibitor.

9. The method according to claim 7 or 8, wherein the pluripotent stem cells are induced pluripotent stem (iPS) cells.

10. A renal interstitial progenitor cell produced by the method according to any one of claims 1 to 9.

11. A pharmaceutical composition comprising the renal interstitial progenitor cell according to claim 10.

12. The pharmaceutical composition according to claim 11, further comprising a nephron progenitor cell.

13. A method of producing renal EPO (erythropoietin)-producing cells, the method comprising the step of culturing renal interstitial progenitor cells in a medium comprising a GSK-3β inhibitor, a SHH signal activator, and a HIF (hypoxia-inducible factor) inhibitor, to produce the renal EPO-producing cells.

14. The method of producing renal EPO-producing cells according to claim 13, wherein the medium further comprises a TGFβ inhibitor and/or retinoic acid.

15. The method of producing renal EPO-producing cells according to claim 13 or 14, wherein the renal interstitial progenitor cells are renal interstitial progenitor cells obtained by the method according to any one of claims 1 to 9.

16. A method of producing renin-producing cells, the method comprising the step of culturing renal interstitial progenitor cells in a medium comprising a GSK-3β inhibitor and a TGFβ inhibitor, but not comprising an SHH signal activator, to produce the renin-producing cells.

17. The method of producing renin-producing cells according to claim 16, wherein the renin-producing cells are mesangial cells or juxtaglomerular cells.

18. The method of producing renin-producing cells according to claim 16 or 17, wherein the medium further comprises BMP, VEGF (vascular endothelial growth factor), and/or retinoic acid.

19. The method of producing renin-producing cells according to any one of claims 16 to 18, wherein the medium further comprises a HIF inhibitor.

20. The method of producing renin-producing cells according to any one of claims 16 to 19, wherein the renal interstitial progenitor cells are renal interstitial progenitor cells obtained by the method according to any one of claims 1 to 9.
